(19) Europäisches Patentamt
European Patent Office
Office européen des brevets

(11) Veröffentlichungsnummer: **0 114 604**
**B1**

(12) # EUROPÄISCHE PATENTSCHRIFT

(45) Veröffentlichungstag der Patentschrift: **30.04.86**

(51) Int. Cl.⁴: **C 07 C 25/13**, C 07 C 17/12, C 07 C 17/38

(21) Anmeldenummer: **84100135.7**

(22) Anmeldetag: **09.01.84**

(54) **Kernhalogenierte 3-Fluortoluole und Verfahren zu ihrer Herstellung.**

(30) Priorität: **21.01.83 DE 3301868**

(43) Veröffentlichungstag der Anmeldung: **01.08.84 Patentblatt 84/31**

(45) Bekanntmachung des Hinweises auf die Patenterteilung: **30.04.86 Patentblatt 86/18**

(84) Benannte Vertragsstaaten: **CH DE FR GB IT LI**

(56) Entgegenhaltungen:
**DE - B - 2 259 870**

**BEILSTEINS HANDBUCH DER ORGANISCHEN CHEMIE, 4. Auflage, 3. Ergänzungswerk, 5. Band, 2. Teil, 1964 SPRINGER-VERLAG, Berlin, Göttingen, Heidelberg Seiten 711, 712**
**PATENTS ABSTRACTS OF JAPAN, unexamines applications, Sektion C, Band 2, Nr. 85, 12. Juli 1978 THE PATENT OFFICE JAPANESE GOVERNMENT Seite 1243 C 78**

(73) Patentinhaber: **BAYER AG, Konzernverwaltung RP Patentabteilung, D-5090 Leverkusen 1 Bayerwerk (DE)**

(72) Erfinder: **Naab, Paul, Dr., Schenkstrasse 32b, D-5600 Wuppertal 21 (DE)**
Erfinder: **Preiss, Michael, Dr., Egenstrasse 21, D-5600 Wuppertal 1 (DE)**

**EP 0 114 604 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue, kernhalogenierte 3-Fluortoluole der Formel I und ein chemisch eigenartiges Verfahren zu ihrer Herstellung.

Zum Stand der Technik gehören Monofluortetrabromtoluole wie 2-Fluor-3,4,5,6-tetrabromtoluol, 3-Fluor-2,4,5,6-tetrabromtoluol und 4-Fluor-2,3,5,6-tetrabromtoluol (DE-AS 2 259 870), m-Dihalogenbenzole, z.B. Dichlorbenzol (Patent Abstracts of Japan, Section C, Bd. 2, Nr. 85, 12. 6. 1978, Seite 1243 C 78) und 5-Fluor-2-brom-toluol bzw. α-Fluor-2-brom-toluol, 4-Fluor-3-brom-toluol, 3-Fluor-4-brom-toluol u.a. (Beilstein, 4.Aufl. 3. Ergänzungswerk, 5. Bd. 2. Teil (1964), 711 und 712). Die erfindungsgemäßen Verbindungen sind demgegenüber ebenso neu wie das Verfahren zu ihrer Herstellung.

Diese neuen erfindungsgemäßen Verbindungen, insbesondere diejenigen der Formeln II, III, IV und V, sollen als Zwischenprodukte für die Herstellung von antibakteriellen Arzneimitteln, insbesondere der 1-Cyclopropyl-6-fluor-1,4-dihydro-7-(1-piperazinyl)-4-oxochinolincarbonsäure der Formel VI (siehe DE-OS 30 33 157), verwendet werden.

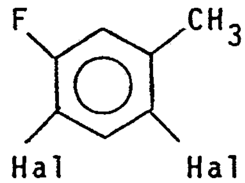

I

Hal: gleich oder verschieden; Cl, Br oder I

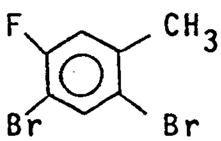

II

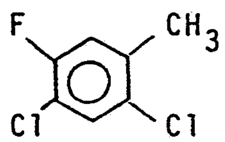

III

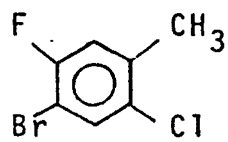

IV

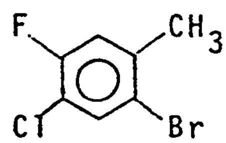

V

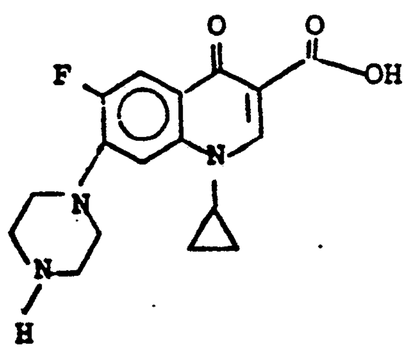

VI

Um eine hohe Reinheit des pharmazeutisch wirksamen Endproduktes zu gewährleisten, müssen schon die Ausgangsverbindungen für die Herstellung dieser Endprodukte ausreichenden Reinheitskriterien entsprechen. Insbesondere muß gewährleistet sein, daß störende Kernisomere nicht mehr vorhanden sind, da ansonsten mit schwierig zu entfernenden Verunreinigungen, geringerer Ausbeute und aufwendigen chemischen Reinigungsoperationen zu rechnen ist.

Es wurde nun gefunden, daß man hoch isomerenreine Verbindungen der Formel I erhält, wenn man 3-Fluortoluol zunächst unter den Bedingungen einer aromatischen Kernsubstitution mit entsprechenden Äquivalenten Halogen oder Halogenierungswittel umsetzt und das entstandene Produkt mit einem Sulfonierungsmittel reagieren läßt.

Überraschenderweise ist bei diesem prozeß die Selektivität außerordentlich hoch. So ist z.B. bei der Browierung von 3-Fluortoluol und enschließender Sulfonierung das Isomerenverhältnis von erwünschtem 3-Fluor-4,6-dibromtoluol zu unerwünschtem 3-Fluor-2,6-dibromtoluol bzw. anderen unerwünschten kernisomeren Dibromderivaten größer als 990:1, wie das gaschromatogramm zeigt. Geeignete Halogene oder Halogenierungsmittel sind alle für diesen Zweck geeigneten Reagenzien, z.B. $Cl_2$, $Br_2$, $I_2$, ICl, IBr, $So_2Cl_2$, $ClSO_3H$,

$ClSO_3F$, Brom in mineralsaurer Lösung, gegebenenfalls in Gegenwart von Katalysatoren, z. B. Essigsäure, Fe, Fe-, Al-, Zn-, Sn- oder Sb-Halogenide, Jod oder Silbersalze (siehe z. B. auch Weygand Hilgetag, org. chem. Experimentalkunst, Johann Ambrosius Barth, Leipzig, S. 146 ff.)

Die bevorzugte Ausführungsform besteht in der Halogenierung mit Halogenen in Gegenwart von Eisenspänen oder Eisensalzen.

Die Halogenierung kann mit oder ohne Lösungsmittel durchgeführt werden, wobei sich eine Reaktion ohne Lösungsmittel der Einfachheit halber anbietet. Die Reaktion kann bei 0° C, tieferer Temperatur, Raumtemperatur oder erhöhter Temperatur durchgeführt werden, je nach Reaktivität und Stärke des Katalysators, wobei jedoch Reaktionen bei 0°C oder besonders bei Raumtemperatur bevorzugt werden.

Geeignete Sulfonierungsmittel sind alle bekannten Reagenzien dieser Art, sofern sie nur reaktiv genug sind. Insbesondere zu nennen sind Schwefelsäure, $SO_3$-Addukte, Oleum oder $SO_3$, wobei Oleum besonders bevorzugt ist. (siehe z.B. auch Weygand Hilgetag, org. chem. Experimentalkunst, Johann Ambrosius Barth, S. 630 ff.)

Die Sulfonierung kann mit oder ohne Lösungsmittel durchgeführt werden, wobei sich eine Reaktion ohne Lösungsmittel der Einfachheit halber anbietet. Die Reaktion kann bei 0° C, tieferer Temperatur, Raumtemperatur oder erhöhter Temperatur durchgeführt werden, je nach Reaktivität des Sulfonierungsmittels, wobei jedoch Reaktionen bei 0°C oder Raumtemperatur bevorzugt werden.

Als geeignete Aufarbeitung bietet sich jede Methode an, die es erlaubt, die Sulfonsäure der entsprechenden kernisomeren Verbindungen von den Verbindungen der Formel I abzutrennen.

Als bevorzugt sei hierbei die Umsetzung mit Basen, wie z.E. Alkalilaugen oder Carbonaten oder Bicarbonaten, genannt. Eine andere geeignete Methode besteht darin, die Sulfonsäure mit einem geeigneten Mittel, z.B. einem niedrig siedenden Kohlenwasserstoff, in dem I löslich ist, auszufällen. Auch eine Kombination aus beiden Methoden ist geeignet.

Um die erfindungsgemäßen Verbindungen in antibakteriell wirksame Stoffe zu überführen, geht man wie folgt vor:

Die erfindungsgemäßen Verbindungen werden in der Seitenkette trihalogeniert und dann zu den Säurechloriden (1) partiell hydrolysiert. Diese werden wie in EP 0 004 279 beschrieben, mit 3-Cyclopropylaminoacrylsäuremethylester in Gegenwart eines basischen Mittels zu einem Acylzwischenprodukt umgesetzt, das zum Chinolonderivat cyclisiert wird. Zugabe von Wasser zum Cyclisierungsgemisch liefert die Chinoloncarbonsäure (2). Deren Umsetzung mit Piperazin führt zu dem gewünschten antibakteriell wirksamen Mittel (3). Die Reaktionssequenz wird durch das nachstehende Formelschema veranschaulicht:

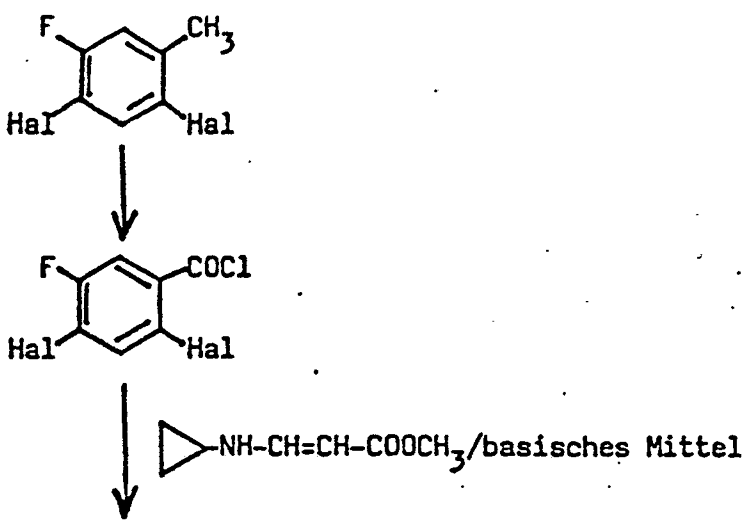


(1)

(2)

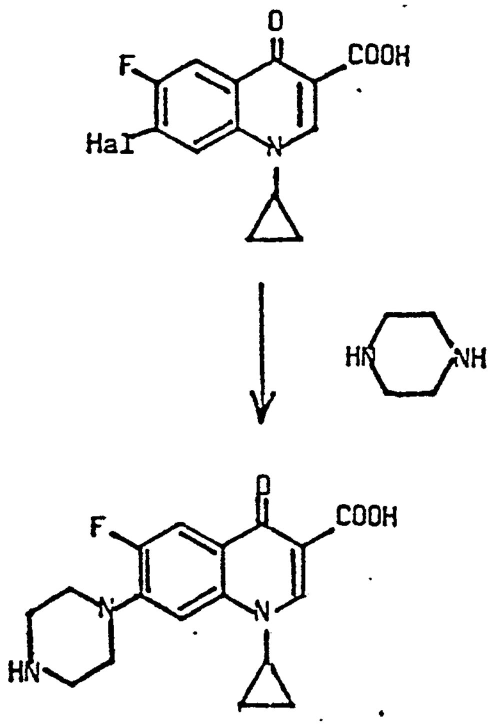

(3)

**Beispiel 1: 3-Fluor-4-brom-6-chlortoluol**

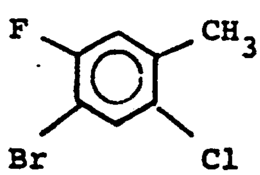

110,1 Gewichtsteile 3-Fluortoluol und 2,2 Gewichtsteile Eisenstaub werden auf 0-5° C gekühlt und innerhalb von ca. 1 3/4 Stunden werden bei der gleichen Temperatur 71 Gewichtsteile Chlor eingeleitet. Anschließend verdrängt man die Salzsäure mit Stickstoff, gibt 2 Gewichtsteile Eisenstaub hinzu und tropft innerhalb von 50 Minuten 52,1 Volumenteile Brom ein.

Nach einer Nachrührzeit von 15 Minuten werden 150 Volumenteile Dichlormethan hinzugefügt, die Lösung filtriert und der Filterrückstand mit 70 Volumenteilen Dichlormethan nachgespült.

Die organische Phase wird mit I80 Volumenteilen Wasser, dann 2 x mit je 120 Volumenteilen 10 %iger Natronlauge und 2 x mit je 150 Volumenteilen Wasser gewaschen, mit 60 Gewichtsteilen Natriumsulfat getrocknet und im Vakuum eingeengt.

Zum Rückstand (204 Gewichtsteile) werden bei 7-10° C 100 Gewichtsteile Oleum (20 %ig) getropft und das Gemisch über Wacht bei Raumtemperatur gerührt.

Aufarbeitung erfolgt durch Eingießen in 213 Gewichtsteile Eis/70 Gewichtsteile Wasser, Extrahieren mit Dichlormethan (3 x je 70 Volumenteile), Waschen der gesamten organischen Phasen mit gesättigter Natriumhydrogencarbonatlösung (2 x je 70 Volumenteile), Filtrieren und Einengen im Vakuum. Der Rückstand

(154,3 Gewichtsteile) wird im Wasserstrahlvakuum destilliert: 144,2 Gewichtsteile ($Kp_{22\ mbar}$: 100-103°C), farbloses Öl (64,5 % der Theorie). Gehalt nach GC 96,8 %. 1H.NMR (DMF-$d_7$; 250 MHz, mit DMF bei $\delta$ = 2.74 als internen Standard):$\delta$ = 2,30 (s, 3H);$\delta$ = 7.28 (d, J= 9Hz, 1H);$\delta$ = 7.58 (d, J ≈ 6Hz, IH).

**Beispiel 2: 3-Fluor-4,6-dibromtoluol**

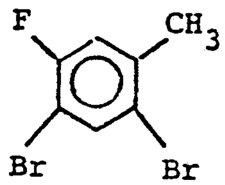

Bei Raumtemperatur werden 110, 1 Gewichtsteile 3-Fluor-toluol und 4 Gewichtsteile Eisenstaub innerhalb von 75 Minuten mit 102,4 Volumenteilen Brom umgesetzt. Anschließend verdrängt man die Bromwasserstoffsäure mit Stickstoff, versetzt mit 150 Volumenteilen Dichlormethan, filtriert und wäscht den Filterrückstand mit 100 Volumenteilen Dichlormethan.

Aufarbeitung erfolgt durch Waschen mit Wasser (2 x je 180 Volumenteile),

10 %iger Natronlauge (2 x je 120 Volumenteile) Wasser (2 x je 150 Volumenteile), Trocknen mit Natriumsulfat (60 Gewichtsteile), Filtrieren und Einengen der Lösung. Der Rückstand (257,8 Gewichtsteile) wird im Wasserstrahlvakuum destilliert: 241,5 Gewichtsteile eines farblosen Öls.

133,9 Gewichtsteile dieses Destillats werden wie im Beispiel 1 beschrieben mit 50 Gewichtsteilen Oleum umgesetzt. Nach Destillation ergeben sich 94,7 Gewichtsteile 3-Fluor-4,6-dibromtoluol (63,7 % der Theorie bezogen auf 3-Fluortoluol). Gehalt nach GC: 99,4 %.

1H-NMR (DMF-$d_7$; 250 MHz; mit DMF bei $\delta$ = 2.74 als internen Standard): $\delta$= 2,33 (s, 3H;$\delta$ = 7,38 (d, J≈9Hz, IH); $\delta$ = 7,84 (d, J≈6Hz, IH).

**Beispiel 3: 3-Fluor-4,6-dibromtoluol**

Die Reaktion wird wie im Beispiel 2 beschrieben durchgeführt, jedoch wird der Rückstand aus der Bromierung direkt mit Oleum umgesetzt. Ausbeute nach Aufarbeitung 76 % der Theorie, GC: 99,2 %ig, und Destillation 71 % der Theorie, Gehalt nach GC: 99,6 %.

**Beispiel 4: 3-Fluor-4,6-dibromtoluol**

Bei 15 - 30 °C werden 50 Gewichtsteile 3-Fluortoluol und 0,45 Gewichtsteile Eisenstaub mit 149,6 Gewichtsteilen Brom umgesetzt. Anschließend verdrängt man die Bromwasserstoffsäure mit Stickstoff, filtriert vom Katalysatorrückstand ab und wäscht das Filter mit 5 Volumenteilen Methylenchlorid nach. Die so erhaltene Lösung wird mit 49,9 Gewichtsteilen Oleum (20%ig) über Nacht umgesetzt. Die Aufarbeitung erfolgt durch Einrühren in 103 Gewichtsteile Eis, Zugeben von 50 Volumenteilen Dichlormethan, Filtrieren und Abtrennen der organischen Phase. Die wässrige Phase wird nochmals mit 34 Volumenteilen Dichlormethan ausgerührt und die organische Phase abgetrennt. Die gesamten organischen Phasen werden danach destilliert. Ausbeute: 91,5 Gewichtsteile Fluordibromtoluol (Gehalt nach GC: 99,5 %).

**Beispiel 5: 3-Fluor-4,6-dibromtoluol**

Die Reaktion wird wie im Beispiel 4 beschrieben durchgeführt, jedoch wird anstelle von Dichlormethan Cyclohexan verwendet. Ausbeute nach Destillation: 90,7 Gewichtsteile (Gehalt nach GC: 99,4 %).

**Beispiel 6: 3-Fluor-4,6-dibromtoluol**

Die Reaktion wird wie im Beispiel 4 beschrieben durchgeführt, jedoch wird nur 3 Stunden mit Oleum umgesetzt. Ausbeute nach Destillation: 90,9 Gewichtsteile (Gehalt nach GC: 99,5 %).

**Beispiel 7: 3-Fluor-4,6-dibromtoluol**

Die Reaktion wird wie im Beispiel 4 beschrieben durchgeführt, jedoch werden anstelle von Oleum (20%ig) 14 Gewichtsteile Oleum (65%ig) genommen. Ausbeute: 90 Gewichtsteile (Gehalt nach GC: 99,6 %).

**Beispiel 8: 3-Fluor-4,6-dibromtoluol**

Die Reaktion wird wie im Beispiel 4 beschrieben durchgeführt, jedoch wird anstelle von Eisenpulver Eisen(III)chlorid als Katalysator verwendet. Ausbeute: 90 Gewichtsteile (Gehalt nach GC: 99,5 %).

**Patentansprüche:**

1. Kernhalogenierte 3-Fluortoluole der allgemeinen Formel

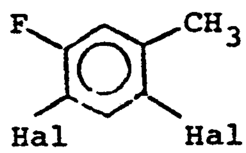

I

in der Hal gleich oder verschieden sein kann und Chlor, Brom oder Jod bedeutet.

2. 3-Fluor-4,6-dibromtoluol.

3. 3-Fluor-4,6-dichlortoluol.

4. 3-Fluor-4-brom-6-chlortoluol.

5. 3-Fluor-4-chlor-6-bromtoluol.

6. Verfahren zur Herstellung von kernhalogenierten 3-Fluortoluolen der allgemeinen Formel I in Anspruch 1, dadurch gekennzeichnet, daß man 3-Fluor-toluol unter den Bedingungen einer aromatischen Kernsubstitution mit entsprechenden Äquivalenten Halogen oder Halogenierungsmittel, gegebenenfalls in Gegenwart von Katalysatoren, umsetzt und das entstandene Produkt mit einem Sulfonierungsmittel umsetzt.

7. Verfahren nach Anspruch 6, dadurch gekennzeichnet, daß man die Halogenierung mit Halogen in Gegenwart von Eisenspänen durchführt.

8. Verfahren nach Anspruch 6, dadurch gekennzeichnet, daß man als Sulfonierungsmittel Oleum verwendet.

**Claims**

1. 3-fluorotoluenes halogenated in the nucleus, of the general formula

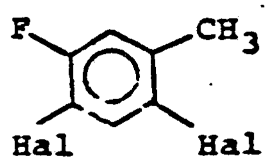

I

in which Hal can be identical or different and denote chlorine, bromine or iodine.

2. 3-fluoro-4,6-dibromotoluene.

3. 3-fluoro-4,6-dichlorotoluene.

4. 3-fluoro-4-bromo-6-chlorotoluene.

5. 3-fluoro-4-chloro-6-bromotoluene.

6. Process for the preparation of 3-fluorotoluenes halogenated in the nucleus, of the general formula I in Claim 1, characterised in that 3-fluorotoluene is reacted, under the conditions of aromatic nuclear substitution, with appropriate equivalents of halogen or halogenating agent, optionally in the presence of catalysts, and the resulting product is reacted with a sulphonating agent.

7. Process according to Claim 6, characterised in that the halogenation is carried out with halogen in the presence of iron turnings.

8. Process according to Claim 6, characterised in that oleum is used as the sulphonating agent.

**Revendications**

1. 3-fluorotoluènes halogénés dans le noyau répondant à la formule générale

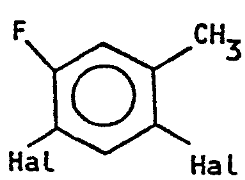

I

FIG9/35

dans laquelle les symboles Hal, ayant des significations identiques ou différentes, représentent le chlore, le brome ou l'iode.

2. Le 3-fluoro-4,6-dibromotoluène.

3. Le 3-fluoro-4,6 dichlorotoluène.

4. Le 3-fluoro-4-bromo-6-chlorotoluène.

5. Le 3-fluoro-4-chloro-6-bromotoluène.

6. Procédé de préparation des 3-fluorotoluènes halogénes dans le noyau de formule générale I de la revendication 1, caractérisé en ce que l'on fait réagir le 3-fluorotoluene dans les conditions d'une substitution du noyau aromatique avec la quantité équivalente correspondante d'halogène ou d'un agent halogénant, éventuellement en presence de catalyseurs, et on fait rèagir le produit obtenu avec un agent sulfonant.

7. Procédé selon la revendication 6, caractérisé en ce que l'halogénation est effectuée à l'aide d'un halogène en présence de copeaux de fer.

8. Procédé selon la revendication 6, caractérisé en ce que l'agent sulfonant utilisé est l'oléum.